(19) Europäisches Patentamt
European Patent Office
Office européen des brevets

(11) Veröffentlichungsnummer: **0 131 845**
**B1**

(12) # EUROPÄISCHE PATENTSCHRIFT

(45) Veröffentlichungstag der Patentschrift: **02.09.87**

(51) Int. Cl.⁴: **A 01 N 43/64,** A 01 N 43/50

(21) Anmeldenummer: **84107728.2**

(22) Anmeldetag: **04.07.84**

(54) **Verwendung von substituierten Diazolyl-alkyl-carbinolen zur Bekämpfung von Pilzen im Pflanzenschutz.**

(30) Priorität: **13.07.83 DE 3325313**

(43) Veröffentlichungstag der Anmeldung: **23.01.85 Patentblatt 85/4**

(45) Bekanntmachung des Hinweises auf die Patenterteilung: **02.09.87 Patentblatt 87/36**

(84) Benannte Vertragsstaaten: **AT BE CH DE FR GB IT LI NL SE**

(56) Entgegenhaltungen:
**EP-A-0 044 605**
**EP-A-0 120 276**
**EP-A-0 122 056**
**EP-A-0 122 693**
**GB-A-2 099 818**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Elbe, Hans- Ludwig, Dr., Dasnöckel 59, D-5600 Wuppertal 11 (DE)**
Erfinder: **Regel, Erik, Dipl.- Ing., Untere Bergerheide 26, D-5600 Wuppertal 1 (DE)**
Erfinder: **Büchel, Karl Heinz, Prof. Dr., Dabringhausener Strasse 42, D-5093 Burscheid (DE)**
Erfinder: **Reinecke, Paul, Dr., Lessingstrasse 11, D-5090 Leverkusen (DE)**
Erfinder: **Brandes, Wilhelm, Dr., Eichendorffstrasse 3, D-5653 Leichlingen (DE)**
Erfinder: **Hänssler, Gerd, Dr., Heymannstrasse 40, D-5090 Leverkusen (DE)**

EP 0 131 845 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft die Verwendung von substituierten Diazolyl-alkyl-carbinolen zur Bekämpfung von Pilzen im Pflanzenschutz.

Es ist bereits bekannt geworden, daß bestimmte Diazolyl-Derivate, wie beispielsweise 1,3-Di-(1,2,4-triazol-1-yl)-2-(2-chlorphenyl)-bzw.-(3-chlorphenyl)- bzw. -(4-chlor-phenyl)- bzw. -phenyl-2-propanol, fungizide Eigenschaften aufweisen (vergleiche EP-A- 0 044 605). Die Wirkung dieser Verbindungen ist jedoch, insbesondere bei niedrigen Aufwandmengen und -konzentrationen, nicht immer ganz befriedigend.

In der EP-A- 0 120 276 werden antimykotisch wirksame substituierte Diazolylalkyl-carbinole offenbart wie sie auch im vorliegenden Fall beschrieben werden. Ein Einsatz dieser Stoffe gegen phytopathogene Pilze wird in der EP-A 0- 120 276 aber nicht erwähnt.

Ferner sind aus der EP-A- 0 122 056 und der EP-A- 0 122 693 fungizid wirksame substituierte Diazolylalkyl-carbinole bekannt, in denen das Carbinol-Kohlenstoffatom mit einem Phenyl-Rest verbunden ist, der gegebenenfalls durch Halogen, Alkyl, Alkoxy und/oder Halogenalkyl substituiert ist. Entsprechende Verbindungen, in denen der betreffende Phenyl-Rest andere als die genannten Gruppen als Substituenten enthält, werden aber nicht erwähnt.

Bei der EP-A- 0 120 276, der EP-A- 0 122 056 und der EP-A- 0 122 693 handelt es sich um Dokumente, die zum Stande der Technik gemäß Artikel 54 (3) EPÜ gehören.

Es wurde gefunden, daß die substituierten Diazolylalkylcarbinole der Formel

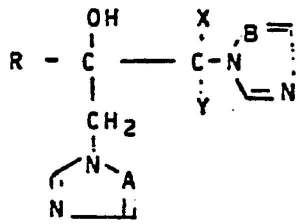

(I)

in welcher

A für ein Stickstoffatom oder die CH-Gruppe steht,

B für ein Stickstoffatom oder die CH-Gruppe steht,

X für Wasserstoff und geradkettiges oder verzweigtes Alkyl mit 1 bis 4 Kohlenstoffatomen steht;

Y für geradkettiges oder verzeigtes Alkyl mit 1 bis 4 Kohlenstoffatomen steht; sowie für den Fall, daß X für Wasserstoff steht, auch für Allyl, Methylallyl, Propargyl, Methylpropargyl oder für gegebenenfalls durch Fluor, Chlor, Brom. Methyl, Isopropyl, tert. Butyl, Methoxy, Methylthio. Trifluormethyl, Trirluormethoxy, Trifluormethylthio, Nitro und Cyano im Phenylteil substituiertes Benzyl steht;

R für Phenyl steht, das einfach oder zweifach, gleich oder verschieden substituiert ist durch Methylthio, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl, sowie durch jeweils gegebenenfalls durch Fluor, Chlor, Methyl substituiertes Phenyl, Phenoxy, Benzyl oder Benzyloxy,

und

R ferner für die Gruppierung

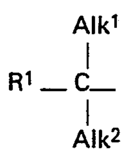

steht,

wobei

$Alk^1$ für Methyl oder Ethyl steht;

$Alk^2$ für Methyl oder Ethyl steht;

$Alk^1$ und $Alk^2$ gemeinsam mit dem Kohlenstoffatom, an das sie gefunden sind, für Cyclobutyl, Cyclopentyl oder Cyclohexyl stehen; und

$R^1$ für Methyl, Ethyl, n-Propyl, i-Propyl, n-Butyl, Neopentyl, sowie für Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei jeder der elf zuvor genannten Reste im Phenylteil substituiert sein kann durch Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Hydroximinomethyl, 1-Hydroximioethyl, Methoximinomethyl, 1-Methoximinoethyl, sowie durch jeweils gegebenenfalls durch Fluor, Chlor, Methyl substituietes Phenyl, Phenoxy, Benzyl und/oder Benzyloxy, sowie deren Säureadditions-Salze und Metallsalz-Komplexe gut zur Bekämpfung von Pilzen im Pflanzenschutz geeignet sind.

Die Verbindungen der Formel (I) besitzen gegebenenfalls zwei asymmetrische Kohlenstoffatome; sie können

dann in zwei geometrischen Isomerenformen vorliegen.

Überraschenderweise zeigen die erfindungsgemäß zu verwendenden substituierten Diazolylalkyl-carbinole der Formel (I) eine bessere Wirksarmkeit gegen phytopathogene Pilze als die aus dem Stand der Technik bereits bekannten Diazolyl-Derivate, wie beispielsweise 1,3-Di-(1,2,4-triazol-1-yl)-2-(2-chlor-phenyl)- bzw. -(3-chlorphenyl)- bzw. -(4-chlorphenyl)- bzw. -phenyl-2-propanol, welche konstitutionell und wirkungsmäßig naheliegende Verbindungen sind. Die erfindungsgemäße Verwendung der Stoffe der Formel (I) stellt somit eine Bereicherung der Technik dar.

Die erfindungsgemäß zu verwendenden substituierten Diazolylalkyl-carbinole sind durch die Formel (I) allgemein definiert. Sie unterscheiden sich von den Verbindungen, die in der EP-A- 0 122 056 und der EP-A- 0 122 693 beschrieben sind, bezüglich der Bedeutungen desjenigen Substituenten, der außer den beiden Diazolylalkyl-Gruppen am Carbinol-Kohlenstoffatom enthalten ist.

Erfindungsgemäß verwendbare Verbindungen sind auch Additionsprodukte aus Säuren und denjenigen substituierten Diazolylalkyl-carbinolen der Formel (I), in denen die Substituenten A, B, X, Y und R die Bedeutungen haben, die bereits für diese Substituenten genannt wurden.

Zu den Säuren die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Erfindungsgemäß verwendbare Verbindungen sind auch Additionsprodukte aus Salzen von Metallen der II. bis IV. Haupt- und der I. und II. sowie IV. bis VIII. Nebengruppe und denjenigen substituierten Diazolylalkyl-carbinolen der Formel (I), in denen die Substituenten A, B, X, Y und R die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen Solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, Salpetersäure und Schwefelsäure. Die erfindungsgemäß zu verwendenden Wirkstoffe sind Gegenstand der EP-A- 0 120 276 und werden erhalten, indem man Azolyloxirane der Formel

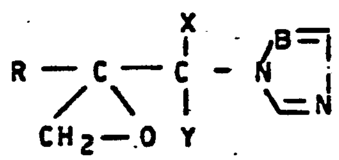

(II)

in welcher

B, R, X und Y die oben angegebene Bedeutung haben,

mit Azolen der Formel

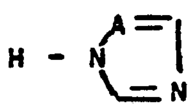

(III)

in welcher

A die oben angegebene Bedeutung hat,

in Gegenwart eines inerten organischen Lösungsmittels, wie beispielsweise Alkohole, und gegebenenfalls in Gegenwart einer Base, wie beispielsweise Alkalimetallalkoholate, bei Temperaturen zwischen 60 und 150°C umsetzt.

An die so erhaltenen Verbindungen der Formel (I) kann gegebenenfalls anschließend eine Säure oder ein Metallsalz addiert werden.

Die Azolyloxirane der Formel (II) sind auch Gegenstand der EP-A- 0 120 276 und können erhalten werden, indem man Azolyl-ketone der Formel

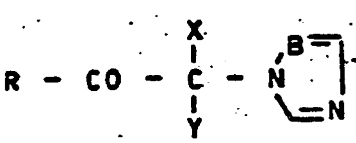

(IV)

in welcher

B, R, X und Y die oben angegebene Bedeutung haben, entweder

α) mit Dimethyloxosulfonium-methylid der Formel

δ + δ -
$(CH_3)_2SOCH_2$ (V)

in an sich bekannter Weise in Gegenwart eines Verdünnungsmittels, wie beispielsweise Dimethylsulfoxid, bei Temperaturen zwischen 20° C und 80° C umsetzt (vgl. hierzu die Angaben in J.Am. Chem.Soc. 87, 1363-1364 (1965)),
oder
β) mit Trimethylsulfonium-methylsulfat der Formel

$[(CH_3)_3S+]\ CH_3SO_4$, (VI)

in an sich bekannter Weise in Gegenwart eines inerten organischen Lösungsmittels, wie Acetonitril, uund in Gegenwart einer Base, wie z. B. Natriummethylat, bei Temperaturen zwischen 0° C bis 60° C, vorzugsweise bei Raumtemperatur, umsetzt (vgl.auch die Angaben in Heterocycles 8, 397 (1977)).
Die so erhaltenen Oxirane der Formel (II) können gegebenenfalls ohne Isolierung direkt weiter umgesetzt werden.
Die Azolyl-ketone der Formel (IV) sind bekannt (vgl. beispielsweise DE-A- 24 31 407, DE-A- 26 10 022, DE-A 26 28 470 und DE-A- 30 48 266 bzw. sind auch sie Gegenstand der EP-A- 0 120 276 und können nach den dort angegebenen Verfahren erhalten werden, indem man z. B. Halogenketone der Formel

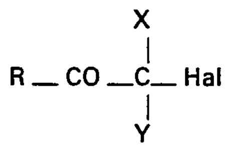
(VII)

in welcher
R, X und Y die oben angegebene Bedeutung haben und
Hal für Chlor oder Brom steht,
mit Azolen der Formel

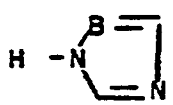
(VIII)

in welcher
B die oben angegebene Bedeutung hat,
in üblicher Weise in Gegenwart eines inerten organischen Lösungsmittels, wie beispielsweise Aceton, und in Gegenwart eines Säurebindemittels, wie beispielsweise Kaliumcarbonat, bei Temperaturen zwischen 40 bis 100° C umsetzt.
Die Halogenketone der Formel (VIII) können in allgemein bekannter Art und Weise erhalten werden, indem man entsprechende Ketone der Formel

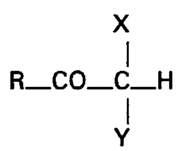
(IX)

in welcher
R, X und y die oben angegebene Bedeutung haben,
mit Chlor oder Brom in Gegenwart eines iherten organischen Lösungsmittels, wie beispielsweise chlorierten Kohlenwasserstoffen, bei Raumtemperatur umsetzt; oder mit üblichen Chlorierungsmitteln, wie beispielsweise Sulfurylchlorid, bei Temperaturen zwischen 20 und 60° umsetzt.
Die erfundungsgemäß verwendbaren Verbindungen der Formel (I) können auch erhalten werden, indem man Diazolyl-ketone der Formel

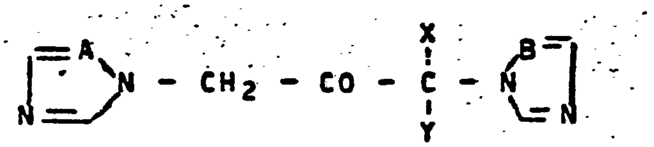

(X)

in welcher
A, B, X und Y die oben angegebene Bedeutung haben,
mit einem Grignard-Reagenz der Formel

R — Mg — -Hal' (XI)

in welcher
R die oben angegebene Bedeutung hat und
Hal' für Halogen steht,
in üblicher Weise unter den Bedingungen einer Grignard-Reaktion umsetzt;
oder indem man Dihalogenalkanole der Formel

OH X
| |
R—C— C Hal
(XII)| |
C $H_2$ Y
|
H al

in welcher
R, Hal, X und Y die oben angegebene Bedeutung haben,
in üblicher Weise mit Azolen der Formel (III) umsetzt.

Die Säureadditionssalze der Verbindungen der Formel können in einfacher Weise nach üblichen Salzbildungsmethoden, z. B. durch Lösen einer Verbindung der Formel (I) in einem geeigneten inerten Lösungsmittel und Hinzufügen der Säure, z.B. Chlorwasserstoffsäure, erhalten werden und in bekannter Weise, z. B. durch Abfiltrieren, isoliert und gegebenenfalls durch Waschen mit einem organischen Lösungsmittel gereinigt werden.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäß zu verwendenden Wirkstoffe weisen eine starke mikrobizide Wirkung auf und können zur Bekämpfung von unerwünschten Mikroorganismen im Pflanzenschutz praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Mittel zur Bekämpfung von phytopathogenen Pilzen können die erfindungsgemäß verwendbaren Wirkstoffe mit besonders gutem Erfolg zur Bekämpfung von Getreidekrankheiten, wie Erysiphe graminis, Cochliobolus sativus, Rost und Pyrenophora teres; von Uromyces-Arten, wie gegen den Erreger des Bohnenrostes (Uromyces appendiculatus); von Venturia-Arten, wie gegen den Erreger des Apfelschorfes (Venturia inaequalis); sowie von Reiskrankheiten, wie Pyricularia oryzae und Pellicularia sasakii, eingesetzt werden.

Hervorzuheben ist, daß die erfindungsgemäß verwendbaren Stoffe nicht nur eine protektive Wirkung entfalten, sondern teilweise auch systemisch sind. So gelingt es, Pflanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestell, z. B. durch Vermischen der Wirkstoffe mit Streckmitteln, also flüssigen Lösungsmitteln, unter Druck stehenden verflüssigten Gasen und/oder festen Trägerstoffen, gegebenen falls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungs mittel als Hilfslösungsmittel verwendet werden. Als flüssige

Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methtylethylketon, Methylisobutylketon oder Cyclohexanon, stark polare Lösungsmittel, wie Dimethylformamid und Dimethylsulfoxid, sowie Wasser; mit verflüssigten gasförmigen Steckmitteln oder Trägerstoffen sind solche Flüssigkeiten gemeint, welche bei normaler Temperatur und unter Normaldruch gasförmig sind, z. B. Aerosol-Treibgase, wie Halogenkohlenwasserstoffe sowie Butan- Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und Fraktionierte natürliche Gesteine wie Calcit, Mamor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier-und/oder schaumerzeugende Mittel kommen in Frage: z. B. nichtionogene und anionische Emulgatoren, wie Polyoxyethylen-Fettsäureester, Polyoxyethylen-Fettalkohol-ether, z. B. Alkylaryl-polyglykol-ether, Alkylsulfonate, Alkylsulfate, Arylsulfonate sowie Eiweißhydrolysate; als Dispergiermittel kommen infrage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischcn 0,1 und 95 Gewichtsprozent Wirkstoff, vorzugsweise zwischen 0,5 und 90 %.

Die erfindungsgemäß verwendbaren Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden. Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0.001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

### Beispiel 1

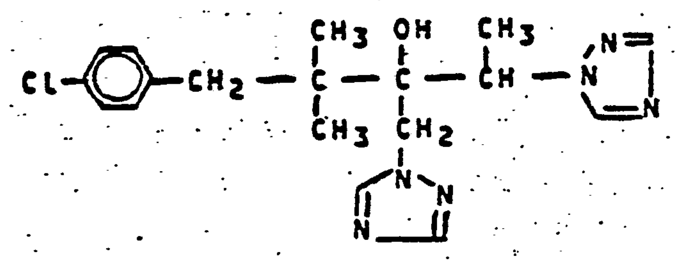

Zu einer Lösung von 0,1 g (0,043 Mol) Natrium in 30 ml n-Propanol werden bei Raumtemperatur unter Rühren 3,6 g (0,052 Mol) 1,2,4-Triazol gegeben. Man versetzt danagh mit einer Lösung von 13,2g (0,043 Mol) 2-(4-Chlorphenyl-tert.-butyl)-2-[1-(1,2,4-triazol-1-yl)-ethyl]oxiran in 20 ml n-Propanol. Das Reaktionsgemisch wird 20 Stunden unter Rückfluß erhitzt, danach abgekühlt und auf 300 ml Wasser gegeben. Man extrahiert mit Methylenchlorid, wäscht die Methylenchloridphase mit Wasser, trocknet über Natriumsulfat und engt im Vakuum ein. Der Rückstand wird säulenchromatographisch (Kieselgel; Essigester: Ether= 3:1) gereinigt. Man erhält 49 (24,8 % der Theorie)-1-(4-Chlorphenyl)-2,2-dimethyl-3-(1,2,4-triazol-1-yl-methyl)-4-(1,2,4-triazol- 1-yl)-3-pentanol vom Schmelzpunkt 59° C.

**Herstellung des Ausgangsproduktes**

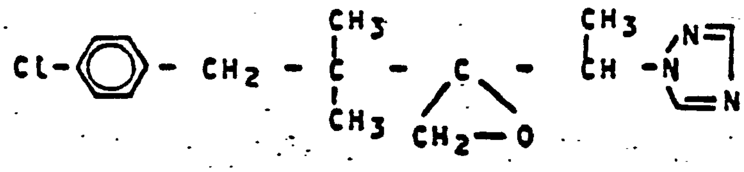

Eine Lösung von 9,8 g (0,077 Mol) Dimethylsulfat und 5,3 g (0,085 Mol) Dimethylsulfid in 50 ml Acetonitril läßt man 5 Tage bei Raumtemperatur rühren. Anschließend läßt man bei Raumtemperatur eine Lösung von 13g (0,044 Mol) (4-Chlor-phenyl-tert.-butyl)-[1-(1,2,4-triazol-1-yl)-ethyl]-keton in 20 ml Acetonitril zutropfen. Bei gleicher Temperatur trägt man 4,8 (0,088 Mol) Natriummethylat ein, läßt 20 Stunden nachrühren und engt anschließend im Vakuum ein. Der Rückstand wird mit einem Gemisch aus 35 ml Essigester und 25 ml Wasser über Nacht verrührt. Die organische Phase wird abgetrennt, über Natriumsulfat getrocknet und im Vakuum eingeengt. Man erhält 13,2 g (98,2 % der Theorie) 2-(4-Chlor-phenyl-tert,-butyl)-2-[1-(1,2,4-triazol-1-yl)-ethyl]-oxiran vom Brechungsindex $n_D^{20} = 1{,}5431$.

In entsprechender Weise werden die folgenden Verbindungen der allgemeinen Formel

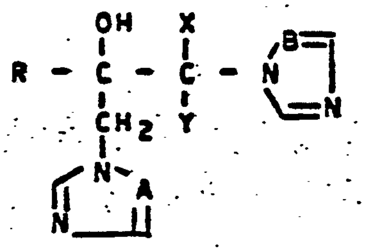

(I)

erhalten:

| Bsp. Nr. | R | X | Y | A | B | Schmelzpunkt (°C) bzw. $n_D^{20}$ |
|---|---|---|---|---|---|---|
| 2 | 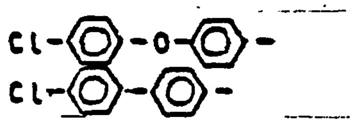 | $CH_3$ | $CH_3$ | N | N | 202 |
| 3 | | $CH_3$ | $CH_3$ | N | N | 185-90 |
| 4 | 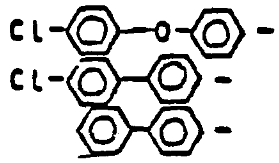 | $CH_3$ | $CH_3$ | CH | N | 90-100 |
| 5 | | $CH_3$ | $CH_3$ | CH | N | 100-10 |
| 6 | | H | $CH_3$ | N | N | glasartig |
| 7 | $(CH_3)_3C-$ | H | $CH_3$ | N | N | 110 |
| 8 | 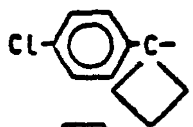 | H | $CH_3$ | N | N | 478 |
| 9 | 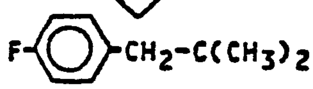 | H | $CH_3$ | N | N | 36 |
| 10 | 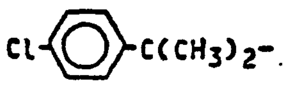 | H | $CH_3$ | N | N | 52 (Form A) |
| 11 | 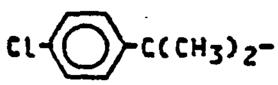 | H | $CH_3$ | N | N | 60 (Form B) |

Formen A und B : Die beiden möglichen geometrischen Isomeren

| | | | | | | |
|---|---|---|---|---|---|---|
| 12 | 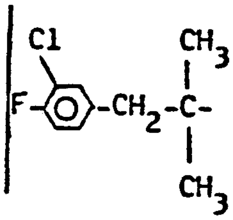 | H | $CH_3$ | N | N | 64-67 |

**Verwendungsbeispiele:**

In den nachfolgenden Verwendungsbeispielen werden die nachstehend angegebenen Verbindungen als Vergleichssubstanzen eingesetzt:

(A) 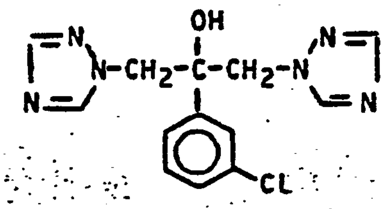

(B) 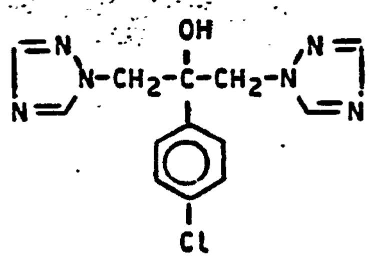

(C) 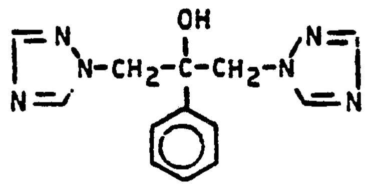

(D) 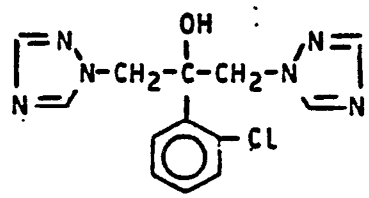

**Beispiel A**

Erysiphe-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 3, 5, 6, 7, 9 und 10.

**Tabelle: A**

Erysiphe-Test (Gerste) / protektiv

| | | | |
|---|---|---|---|
| 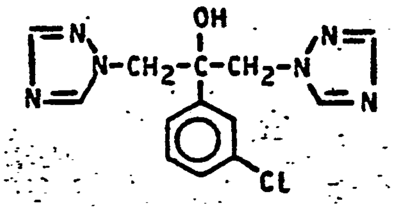 (A) (bekannt) | | 0,0025 | 100 |
| 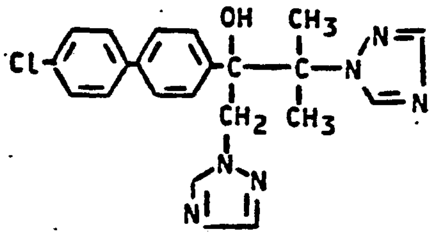 | (3) | 0,0025 | 25,0 |
| 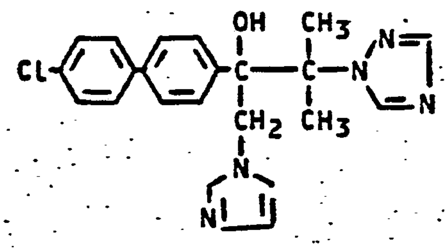 | (5) | 0,0025 | 50,0 |
| 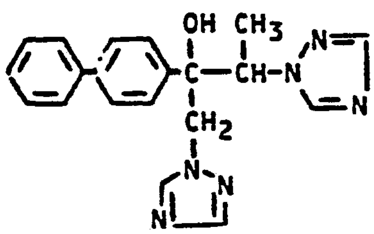 | (6) | 0,0025 | 25,0 |
| 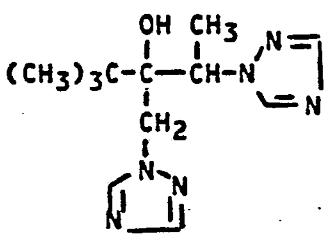 | (7) | 0,0025 | 25,0 |

**Tabelle A** (Fortsetzung)

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 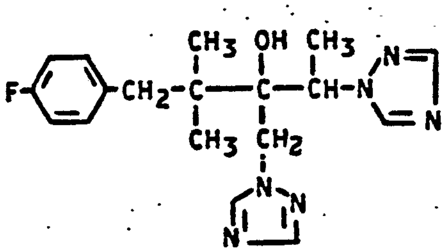 (9) | 0,0025 | 12,5 |
| 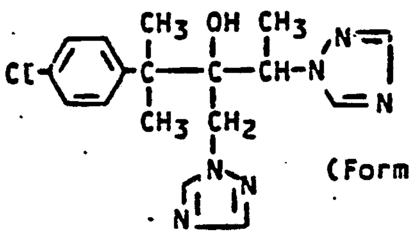 (Form A) (10) | 0,0025 | 25,0 |

**Beispeil: B**

Cochliobolus sativus-Test (Gerste)/protektiv
Lösungsmittel: 100 Gewichtsteile Dimethyllormamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamktit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispie 3, 5, 6, 9 und 10.

**Tabelle B**

Cochliobolus sativus-Test(Gerste) / protektiv

| Wirkstoff | | Wirkstoffkonzentration in der Spritzbrühe in Gew.-% | Krankheitsbefall in % der unbehandelten Kontrolle |
|---|---|---|---|
| 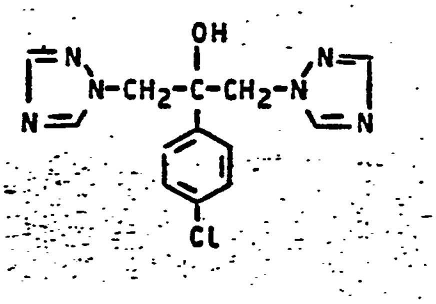<br>(B) (bekannt) | | 0,025 | 100 |
| 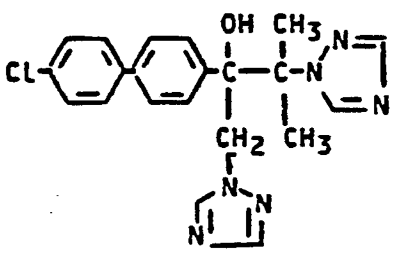 | (3) | 0,025 | 21,2 |
| 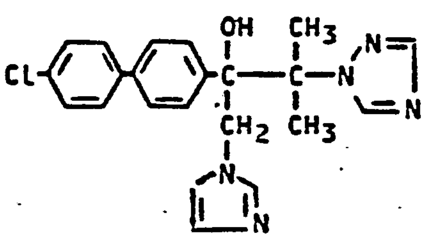 | (5) | 0,025 | 12,5 |
| 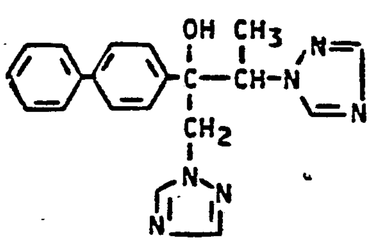 | (6) | 0,025 | 12,5 |

Tabelle B (Fortsetzung)

Cochliobolus sativus-Test(Gerste) / protektiv

| Wirkstoff | | Wirkstoffkonzentration in der Spritzbrühe in Gew.-% | Krankheitsbefall in % der unbehandelten Kontrolle |
|---|---|---|---|
| 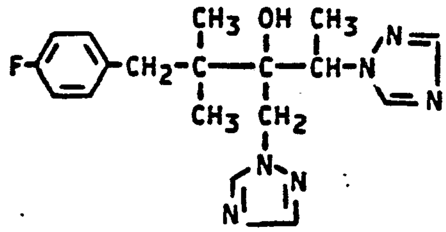 | (9) | 0,025 | 0,0 |
| 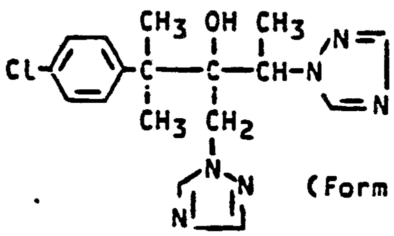 (Form A) | (10) | 0,025 | 33,3 |

**Patentanspruch**

1. Verwendung von substituierten Diazolyalkylcarbinolen der Formel

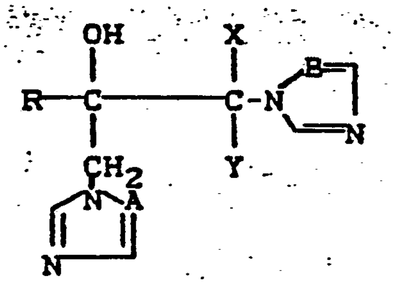

(I)

in welcher

A für ein Stickstoffatom oder die CH-Gruppe steht;

B für ein Stickstoffatom oder die CH-Gruppe steht;

X für Wasserstoff und geradkettiges oder verzweigtes Alkyl mit 1 bis 4 Kohlemstoffatomen steht;

Y für geradkettiges oder verzeigtes Alkyl mit 1 bis 4 Kohlenstoffatomen steht; sowie fü dem Fall, daß X für Wasserstoff steht, auch für Allyl, Methylallyl, Propargyl, Methylpropargyl oder für gegebenenfalls durch Fluor, Chlor, Brom, Methyl, Isopropyl, tert.-Butyl, Methoxy, Methylthio, Trifluormethyl, Trifluormethoxy, Trifluormethylthio, Nitro und Cyano im Phenylteil substituiertes Benzyl steht;

R für Phenyl steht, das einfach oder zweifach, gleich oder verschieden substituiert ist durch Methylthio, Trifluormethoxy, Trifluormethylthio, Nitro, Cyano, Hydroxy, Hydroxycarbonyl, Methoxycarbonyl, Ethoxycarbonyl, Hydroximinomethyl, 1-Hydroximinoethyl, Methoximinomethyl, 1-Methoximinoethyl, sowie durch jeweils gegebenenfalls durch Fluor, Chlor, Methyl substituiertes Phenyl, Phenoxy, Benzyl oder Benzyloxy,

und

R ferner für die Gruppierung

$$R^1-\overset{Alk_1}{\underset{Alk^2}{\overset{|}{\underset{|}{C}}}}-$$

steht,

wobei

$Alk^1$ für Methyl oder Ethyl steht;

$Alk^2$ Eür Methyl oder Ethyl steht;

$Alk^1$ und $Alk^2$ gemeinsam mit dem Kohlenstoffatom, an das sie gebunden sind, für Cyclobutyl, Cyclopentyl oder Cyclohexyl stehen; und

$R^1$ für Methyl, Ethyl, n-Propyl, i-Propyl, n-Butyl, Neopentyl, sowie für Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei jeder der elf zuvor genannten Reste im Phenylteil substituiert sein kann durch Flour, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Hydroximinomethyl, 1-Hydroximioethyl, Methoximinomethyl, 1-Methoximinoethyl, sowie durch jeweils gegebenenfalls durch Fluor, Chlor, Methyl substituiertes Phenyl, Phenoxy, Benzyl und/oder Benzyloxy,

sowie von deren Säureadditions-Salzen und Metallsalz-Komplexen zur Bekämpfung von Pilzen im Pflanzenschultz.

**Revendication**

1. Utilisation de diazolylalkyl-carbinols substitués de formule

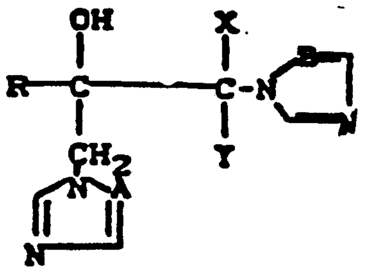

(I)

dans laquelle

A représente un atome d'azote ou le groupe CH,

B représente un atome d'azote ou le groupe CH,

X représente l'hydrogène et un groupe alkyle à chaîne droite ou ramifiée en $C_1$ -$C_4$,

Y représente un groupe alkyle à chaîne droite ou ramifiée en $C_1$ -$C_4$ ou encore, lorsque X représente l'hydrogène, un groupe allyle, méthylallyle, propargyle, méthylpropargyle ou benzyle éventuellement substitué dans la partie phényle par le fluor, le chlore, le brome, des groupes méthyle, isopropyle, tert.butyle, méthoxy, méthylthio, trifluorométhyle, trifluorométhoxy, trifluorométhylthio, nitro et cyano,

R représente un groupe phényle portant 1 ou 2 substituants identiques ou différents choisis parmi les groupes méthylthio, trifluorométhoxy, trifluorométhylthio, nitro, cyano, hydroxy, hydroxycarbonyle, méthoxycarbonyle, éthoxycarbonyle, hydroximinométhyle, 1-hydroximinoéthyle, méthoximinométhyle, 1-méthoximinoéthyle ou phényle, phénoxy, benzyle ou benzyloxy, chacun éventuellement substitué par le fluor, le chlore ou des groupes méthyle, et

R représente en outre le groupement

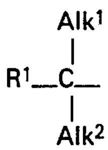

dans lequel

$Alk^1$ représente un groupe méthyle ou éthyle,

$Alk^2$ représente un groupe méthyle ou éthyle,

$Alk^1$ et $Alk^2$ forment ensemble et avec l'atome de carbone auquel ils sont reliés un groupe cyclobutyle, cyclopentyle ou cyclohexyle, et

$R^1$ represente un groupe methyle, ethyle, n-propyle, isopropyle, n-butyle, néopentyle ou encore phényle, benzyle, phénéthyle, phénoxy, phénylthio, phénoxyméthyle, phénoxyéthyle, phénylthiométhyle, phénylthioéthyle, benzyloxy ou benzylthio, chacun des onze groupes mentionnés en dernier pouvant être substitué dans la partie phényle par le fluor, le chlore, le brome, des groupes méthyle, isopropyle, tert.-butyle, méthoxy, méthylthio, trifluorométhyle, trifluorométhoxy, trifluorométhylthio, nitro, cyano, hydroxy, hydroxycarbonyle, hydroximinométhyle, 1-hydroximinoéthyle, méthoximinométhyle, 1-méthoximinoéthyle ou encore phényle, phénoxy, benzyle et/ou benzyloxy, chacun éventuellement substitué par le flour, le chlore ou des groupes méthyle,

et de leurs sels formés par addition avec des acides et complexes de sels métalliques dans la lutte contre les mycètes pour la protection des végétaux.

**Claim**

1. Use of substituted diazolyalkyl carbinols of the formula

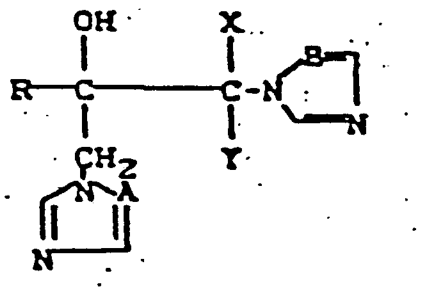

(I)

in which

A represents a nitrogen atom or the CH group;

B represents a nitrogen atom or the CH group;

X represents hydrogen and straight-chain or branched alkyl with 1 to 4 carbon atoms;

Y represents straight-chain or branched alkyl with 1 to 4 carbon atoms; and, if X represents hydrogen, also represents allyl, methylallyl, propargyl, methylpropargyl or benzyl which is optionally substituted in the phenyl part by flourine, chlorine, bromine, methyl, isopropyl, tert.-butyl, methoxy, methylthio, triflouoromethyl, trifluoromethoxy, triflouromethylthio, nitro and cyano;

R represents phenyl which is mono- or disubstituted by identical or different substituents from the group comprising methylthio, triflouromethoxy, trifluoromethylthio, nitro, cyano, hydroxyl, hydroxycarbonyl, methoxycarbonyl, ethoxycarbonyl, hydroximinomethyl, 1-hydroximinoethyl, methoximinomethyl and 1-methoximinoethyl, and also comprising phenyl, phenoxy, benzyl or benzyloxy, each of which is optionally substituted by flourine, chlorine or methyl,

and

R furthermore represents the grouping

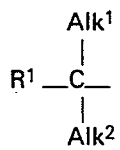

wherein

$Alk^1$ represents methyl or ethyl,

$Alak^2$ represents methyl or ethyl;

$Alk^1$ and $Alk^2$, together with the carbon atom to which they are bonded, represent cyclobutyl, cyclopentyl or cyclohexyl; and

$R^1$ represents methyl, ethyl, n-propyl, i-propyl, n-butyl, neopentyl, and phenyl, benzyl, phenethyl, phenoxy, phenylthio, pnenoxymethyl, phenoxyethyl, phenylthiomethyl, phenylthioethyl, benzyloxy or benzylthio, it being possible for each of the eleven aforementioned radicals to be substituted in the phenyl part by fluorine, chlorine, bromine, methyl, isopropyl, tert.-butyl, methoxy, methylthio, trifluoromethyl, trifluoromethoxy, trifluoromethylthio, nitro, cyano, hydroxyl, hydroxycarbonyl, hydroximinomethyl, 1-hydroximinoethyl, methoximinomethyl or 1-methoximinoethyl, and by phenyl, phenoxy, benzyl and/or benzyloxy, each of which is optionally substituted by fluorine, chlorine or methyl,

and acid addition salts and metal salt complexes thereof for combating fungi in plant protection.